# EUROPEAN PATENT SPECIFICATION

(11) **EP 3 185 157 B1**
(45) Date of publication and mention of the grant of the patent: **08.07.2020**
(21) Application number: 15307130.3
(22) Date of filing: 23.12.2015
(51) Int. Cl.: G16B 30/00, C12N 15/113

(54) **COMPUTER-IMPLEMENTED METHOD FOR THE IDENTIFICATION OF MICRORNAS**
COMPUTERIMPLEMENTIERTES VERFAHREN ZUR IDENTIFIZIERUNG VON MIKRO-RNAS
PROCÉDÉ MIS EN OEUVRE PAR ORDINATEUR POUR L'IDENTIFICATION DE MICRO-ARNS

(43) Date of publication of application: 28.06.2017
(73) Proprietor: Biofacet SAS, 92500 Rueil-Malmaison (FR)
(72) Inventor: CODANI, Jean-Jacques, 92500 RUEIL-MALMAISON (FR); WOZNIAK, Andrzej, 91120 PALAISEAU (FR)
(74) Representative: Vial, Lionel

(56) References cited:
- WO-A2-2006/105436
- CN-A- 103 031 302
- HENDRIX DAVID ET AL: "miRTRAP, a computational method for the systematic identification of miRNAs from high throughput sequencing data", GENOME BIOLOGY, BIOMED CENTRAL LTD., LONDON, GB, vol. 11, no. 4, 6 April 2010 (2010-04-06), page R39, XP021085593, ISSN: 1465-6906, DOI: 10.1186/GB-2010-11-4-R39 & D Hendrix ET AL: "Supplementary Methods - miRTRAP, a computational method for the systematic identification of miRNAs from high throughput sequencing data", Genome Biology, 6 April 2010 (2010-04-06), pages 1-11, XP055277813, Retrieved from the Internet: URL:http://genomebiology.biomedcentral.com /articles/10.1186/gb-2010-11-4-r39 [retrieved on 2016-06-03]
- SUSAN HIGASHI ET AL: "Mirinho: An efficient and general plant and animal pre-miRNA predictor for genomic and deep sequencing data", BMC BIOINFORMATICS, BIOMED CENTRAL, LONDON, GB, vol. 16, no. 1, 29 May 2015 (2015-05-29), page 179, XP021222404, ISSN: 1471-2105, DOI: 10.1186/S12859-015-0594-0
- N. W. BREAKFIELD ET AL: "High-resolution experimental and computational profiling of tissue-specific known and novel miRNAs in Arabidopsis", GENOME RESEARCH, vol. 22, no. 1, 22 September 2011 (2011-09-22), pages 163-176, XP055277439, ISSN: 1088-9051, DOI: 10.1101/gr.123547.111
- WEIYANG SHI ET AL: "A distinct class of small RNAs arises from pre-miRNA-proximal regions in a simple chordate", NATURE STRUCTURAL AND MOLECULAR BIOLOGY, vol. 16, no. 2, 1 February 2009 (2009-02-01), pages 183-189, XP055277978, US ISSN: 1545-9993, DOI: 10.1038/nsmb.1536
- H. S. SOIFER ET AL: "A role for the Dicer helicase domain in the processing of thermodynamically unstable hairpin RNAs", NUCLEIC ACIDS RESEARCH, vol. 36, no. 20, 1 November 2008 (2008-11-01), pages 6511-6522, XP055278041, GB ISSN: 0305-1048, DOI: 10.1093/nar/gkn687
- MATTHIAS HACKL ET AL: "Next-generation sequencing of the Chinese hamster ovary microRNA transcriptome: Identification, annotation and profiling of microRNAs as targets for cellular engineering", JOURNAL OF BIOTECHNOLOGY, vol. 153, no. 1-2, 1 April 2011 (2011-04-01), pages 62-75, XP055094800, ISSN: 0168-1656, DOI: 10.1016/j.jbiotec.2011.02.011

## Description

### Field of the invention

The present invention relates to a computer-implemented method for determining if a nucleic acid sequence belongs to a microRNA (miRNA).

### Technical background

The rise of next generation sequencing (NGS) of small RNA has lead to a significant increase in the discovery of novel microRNAs (miRNAs) over the past years. However, miRNA characterization from NGS reads remains a difficult task since mature forms of the precursor RNA are generally short reads of 18-23 nucleotides (nt). Indeed, not only reads clipping is not 100% accurate, but the short lengths in play can make mapping an arduous task.

Most software approaches rely on sophisticated pipelines chaining mapping, RNA folding and statistical analysis phases. All in all, these approaches essentially rely on the fundamental *dogma* that a RNA-loop is primarily characterized by a computer-aided folding method based on the prediction of the secondary structure of the RNA, the most widely used being that described by Lorenz et al. (2011) Algorithms for Molecular Biology: AMB, 6:26.

Briefly, reads mappers, considering a read as a putative mature miRNA, begin by locating a read on the genome as a perfect match. From this anchor point, the surrounding DNA region is analyzed through its RNA folding angle. Many refinements are then usually performed: z-scoring of folds through randomization processes, probabilistic models of degradation of RNA due to a Dicer-like process, analysis of the presence of other reads in the region, homology with known miRNA-encoding genes (MIRs), and more.

Many tools based on the foregoing have been devised, among which stands the well-established miRDeep (Friedländer et al. (2008) Nature Biotechnology 26:407-415) which has evolved into miRDeep2 (Friedlander et al. (2012) Nucleic Acids Research 40:37-2). Besides, Hendrix et al. (2010) Genome Biology 11:R39, Higashi et al. (2015) BMC Bioinformatics 16:179 and Breakfield et al. (2012) Genome Research 22:163-176 also disclose methods of identifying miRNA candidates.

As previously mentioned, these tools are mostly based on the "perfect match + RNAfolding" principle. This approach faces two problems that compound each other:
*(i)* the ubiquity of short reads leads to a very large number of anchoring regions, and
*(ii)* if folding is powerful on a moderate number of targeted regions, it is hardly applicable at the genome level.

Moreover, rechecking that some star-miRNAs (the less expressed members of the mature miRNA complex) are also present both in the predicted region and in the dataset adds complexity to the analysis.

Accordingly, there is still a need for methods allowing fast and accurate detection of miRNAs.

### Description of the invention

As a foreword, in the following, unless specified otherwise, nucleotide sequences are oriented in the 5' to 3' direction. Besides, when a sequence A is said to "comprise" a sequence B, it will be understood that sequence A can contain other sequences in addition to sequence B. In contrast, when a sequence A is said to "consist of" or to be "constituted of" a sequence B, it will be understood that sequence A cannot contain sequences in addition to sequence B.

The present invention arises from the unexpected discovery, by the present inventors, that first screening the genomic neighborhood of portions of a genomic sequence perfectly aligning with a RNA read for portions aligning with errors with the reverse complementary of the RNA read enables detecting miRNAs with a selectivity and a specificity at least equivalent to method of the prior art while dividing computing time by two orders of magnitude.

Thus, the present invention relates to a computer-implemented method for determining if a nucleic acid sequence belongs to a microRNA (miRNA) candidate encoded by at least one known genomic sequence, comprising:
a) determining if the nucleic acid sequence or the reverse complementary sequence thereof can fully align with a first subsequence of the known genomic sequence;
b) then,
   - if the nucleic acid sequence fully aligns with the first subsequence of the known genomic sequence, determining if the reverse complementary sequence of the nucleic acid sequence can align with a second subsequence of the known genomic sequence with X error(s) at the most, or
   - if the reverse complementary nucleic acid sequence of the nucleic acid sequence fully aligns with the first subsequence of the known genomic sequence, determining if the nucleic acid sequence can align with a second subsequence of the known genomic sequence with X error(s) at the most;
      wherein X is an integer from 0 to 0.6L, wherein L is the length of the nucleic acid sequence, and a third subsequence of the known genomic sequence which consists of the first and second subsequences and of the portion of the known genomic sequence between the first and second subsequences has a length T, wherein T ≤ 3000 nucleotides;
c) whereby, if conditions a) and b) are met, the nucleic acid sequence is determined to belong to a miRNA candidate.

As will be clear to one of skill in the art, it is considered that the following steps a1) and b1) are an equivalent alternative to the above-defined steps a) and b):
a1) determining if the nucleic acid sequence can fully align with a first subsequence of the forward strand of the known genomic sequence or of the reverse strand of the known genomic sequence;
b1) then,
   - if the nucleic acid sequence fully aligns with the first subsequence of the forward strand the known genomic sequence, determining if the nucleic acid sequence can align with a second subsequence of the reverse strand of the known genomic sequence with X error(s) at the most, or
   - if the nucleic acid sequence fully aligns with the first subsequence of the reverse strand of the known genomic sequence, determining if the nucleic acid sequence can align with a second subsequence of the forward strand of the known genomic sequence with X error(s) at the most;
wherein X is an integer from 0 to 0.6L, wherein L is the length of the nucleic acid sequence, and a third subsequence of the known genomic sequence which consists of the first subsequence, the reverse complementary of the second subsequence and of the portion of the known genomic sequence between the first subsequence and the reverse complementary of the second subsequence, or which consists of the reverse complementary of the first subsequence, the second subsequence and of the portion of the known genomic sequence between the reverse complementary of the first subsequence and the second subsequence, has a length T, wherein T ≤ 3000 nucleotides.

However, the latter steps a1) and b1) require storing both strands *(i.e.* the forward strand and the reverse strand) of the known genomic sequence in the memory of the computer, whereas only one strand is needed for steps a) and b).

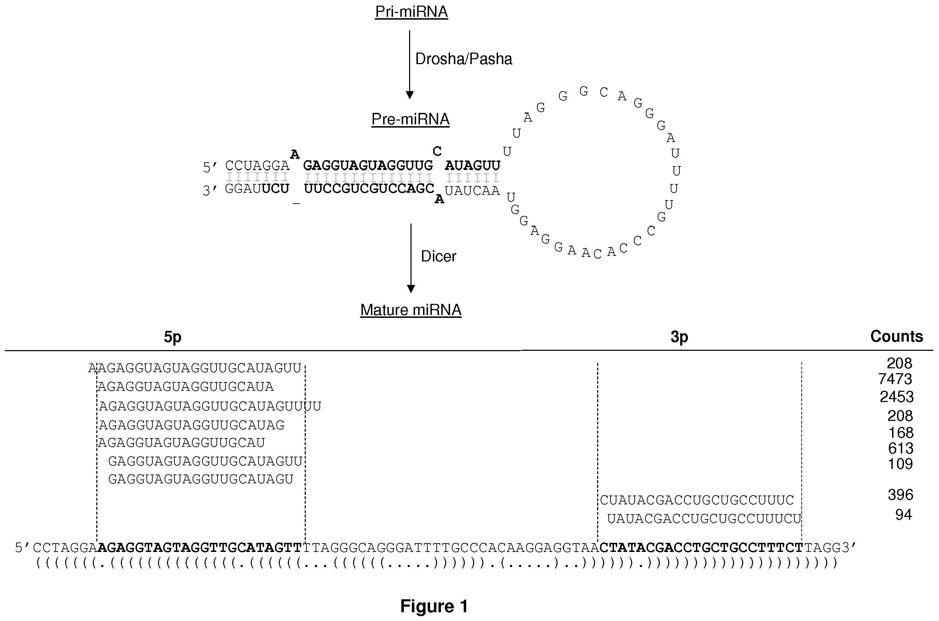

**Figure 1** sums up the biogenesis of micro-RNAs (miRNAs) through the example of Hsa-let-7d. A primary miRNA (pri-miRNA) is transcribed from chromosomal DNA to yield a stem-loop precursor miRNA (pre-miRNA) after the action of Drosha and Pasha. The pre-miRNA is then digested by Dicer to yield the mature miRNA (miR). As is shown in the figure, mature miRNAs originate in the stem of the pre-miRNA. A same pre-miRNA may liberate two matures miRNA sequences, one on the 5' side of the pre-miRNA, before the loop part of the pre-miRNA oriented in the 5' to 3' direction, named with the suffix -5p, and one on the 3' side, after the loop part, named with the suffix -3p. However, a pre-miRNA may also liberate only mature miRNA sequence, either on the 5' side or the 3' side. Besides, it can also be seen that, mainly because of the mode of action of Dicer, the mature miRNA sequences can be found under the form of several length variants varying from a few nucleotides. By convention, the miR-5p and the miR-3p are represented by the variants with the highest level of expression (counts) or by sequences constituted of the nucleotides most found in the variants. Often, one of miR-5p and miR-3p will be significantly more expressed than the other; by convention the less expressed of the two will then be appended a star (*) suffix, e.g. miR Hsa-let-7d*. The portion of the genome aligning with the unfolded pre-miRNA is generally denoted MIR.

As intended herein "determining if a nucleic acid sequence belongs to a microRNA (miRNA) candidate" means determining if the nucleic acid sequence is a putative mature miRNA sequence or a fragment thereof. Thus, the method of the invention also amounts to identifying putative miRNA sequence or miRNA candidates from nucleic acid sequences, e.g. reads. Besides, as will be clear to one of skill in the art, the method according to the invention also amounts to identifying putative MIR sequences.

As intended herein a "known genomic sequence" is a miRNA-encoding sequence. Preferably, the known genomic sequence is a DNA sequence. The known genomic sequence may consist of only one strand, e.g. + or - strand, or of both strands. However, as should be clear to one of skill in the art, when steps a) and b) according to the invention are performed the subsequences of the known genomic sequence are on a same strand of the known genomic sequence. Preferably also the known genomic sequence is a contig, a scaffold or a chromosome. The known genomic sequence can consist of a single individual sequence or several individual sequences. The length of the known genomic sequence can be greater than 10⁵, 10⁶ or 10⁹ nucleotides and smaller than 10¹⁵ or 10¹² nucleotides.

The "nucleic acid sequence" according to the invention is preferably DNA or RNA, more preferably RNA. The nucleic acid sequence according to the invention has a length L, where L is preferably smaller than 100, 75, 50 or 25 nucleotides and preferably greater than 5, 10 or 15 nucleotides; more preferably 18 ≤ L ≤ 23 nucleotides. Preferably also, the nucleic acid sequence is a sequencing read, *i.e.* a read, more preferably a RNA read, in particular a next generation sequencing (NGS) RNA read.

As will be clear to one of skill in the art, the known genomic sequence and the nucleic acid sequence preferably originate from a same species, more preferably from a same organism. Preferably, the known genomic sequence and the nucleic acid sequence are from plants or animals.

It is well within the common skills of one of skill in the art to perform the alignments.. Preferably, the alignment with errors is performed with a method allowing a high percentage of errors on a short sequence which is preferably not homology-based but rather string-matching based, such as the Fast Bit-Vector algorithm for Approximate String Matching (Myers (1999) Journal of the ACM 46:395-41). In contrast, it is preferred to perform a fast mapping method for the full alignment. As intended herein, the expression "fully aligns" is considered synonymous with "perfectly aligns" and means that each nucleotide of the nucleic acid sequence (or the reverse complementary of the nucleic acid sequence), *e.g*. the read, is aligned, *i.e.* matches, with a nucleotide of a first subsequence, *i.e.* portion, of the genomic sequence. As should be clear to one of skill in the art, in the frame of the present invention, U (Uracile), found in RNA sequences, is considered a synonym for T (Thymine), found in DNA sequences. As intended herein, the expression "aligns with errors" means that when the reverse complementary of the nucleic acid sequence (or the nucleic acid sequence) is aligned with a second subsequence of the genomic sequence, at least one nucleotide of the reverse complementary of the nucleic acid sequence (or the nucleic acid sequence) is not matched to at least one nucleotide of the second subsequence or at least one nucleotide of the subsequence is not matched to at least one nucleotide of the reverse complementary of the nucleic acid sequence (or the nucleic acid sequence). The lack of match may be due to the fact that the two aligned nucleotides are different or that a nucleotide is lacking (gapped alignment) in the reverse complementary of the nucleic acid sequence (or in the nucleic acid sequence) or in the second subsequence. Preferably, X ≤ 0.5L or X ≤ 0.4L. Preferably also, X ≤ 6, 7, 8, 9 or 10. Still preferably, X ≥ 1.

Preferably, alignment with the second subsequence in step b) takes wobble pairing *(i.e.* G-U) into account. In other words a C (Cytosine) in the reverse complementary of the nucleic acid sequence (or the nucleic acid sequence) aligned with a T (Thymine) in the second subsequence can be considered as a match and an A (Adenine) in the reverse complementary of the nucleic acid sequence (or the nucleic acid sequence) aligned with a G (Guanine) in the second subsequence can be considered as a match.

As intended herein the "reverse complementary" of nucleic acid sequence is the complementary of the nucleic acid sequence which is reoriented (reversed) in the same direction as the nucleic acid. By way of example, the reverse complementary of 5' AUGC 3' is 5' GCAU 3'.

Preferably 30 ≤ T ≤ 3000, more preferably 30 ≤ T ≤ 350. Preferably also, 350 ≤ T ≤ 3000.

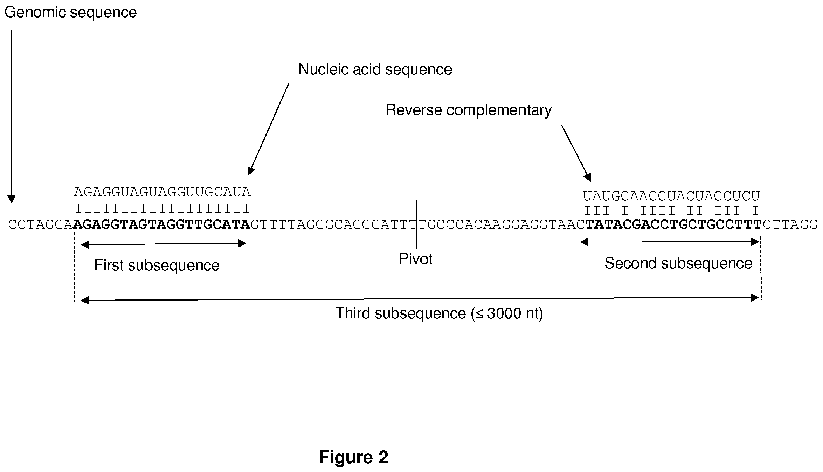

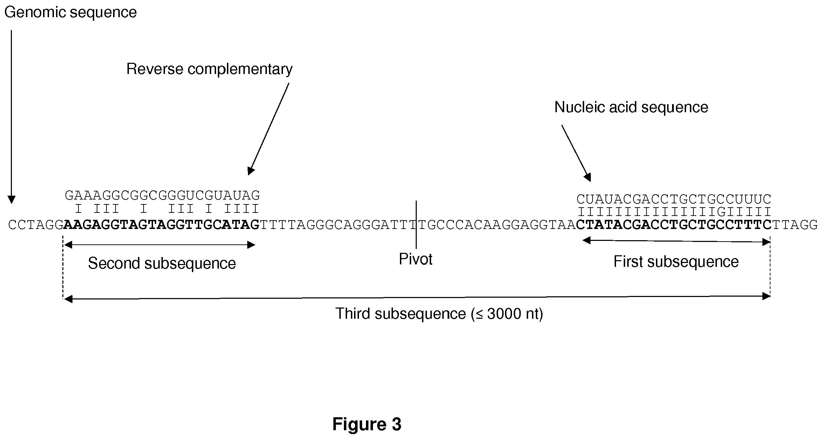

**Figures 2** **and** **3** illustrate the method of the invention with nucleic acid sequences consisting of reads of the most expressed variants respectively of miR Hsa-let-7d-5p and miR Hsa-let-7d-3p.

In a preferred embodiment of the invention, the above-defined method further comprises:
- providing at least two nucleic acid sequences for which conditions a) and b) are met;
- determining that the at least two nucleic acid sequences belong to a same miRNA candidate if their respective third subsequences of the known genomic sequences share a same pivot.

As intended herein, the "pivot" is the "center" of the third subsequence or the point through which passes the folding axis of the secondary structure of the third subsequence so that first and second subsequence are paired. For determining that the third subsequences share a same pivot, one can determine the respective positions of the pivots on the genomic sequence.

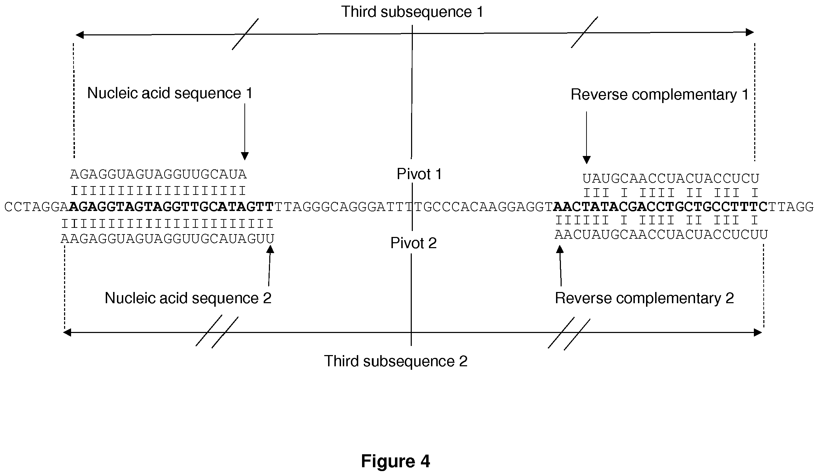

**Figure 4** illustrates the above preferred embodiment of the invention with two nucleic acid sequences (1 and 2) respectively corresponding to reads of the two most expressed variants of Hsa-let-7d-5p. It can be readily seen that the two third subsequences (1 and 2) share a same pivot.

Preferably, in the above embodiment the at least two nucleic acid sequences mutually overlap. This case is notably shown in **Figure 4****.** Preferably, the overlap is of a least 2, 3, 4, 5, 6, 7, 8, 9, 10, 11, 12, 13, 14 or 15 nucleotides. Preferably also, the overlap is of less than 20, 19, 18, 17, 16, or 15 nucleotides.

Preferably also, in the above preferred embodiment one of the at least two nucleic acid sequences fully aligns with a subsequence of the known genomic sequence while the reverse complementary of the other of the at least two nucleic acid sequences fully aligns with a subsequence of the known genomic sequence. This embodiment ensures that nucleic acid sequences, *e.g.* reads, corresponding both to the putative miR-5p and miR-3p are present, increasing the likelihood that the miRNA candidate is indeed a miRNA.

In another preferred embodiment of the invention, the above-defined method further comprises modeling the secondary structure of the third subsequence of the known genomic sequence consisting of the respective first and second subsequences and of the portion of the known genomic sequence between the first and second subsequences by minimizing nucleotide-pairing free-energy. By way of example this can be done by generating a Vienna bracket-dot notation using RNAfold (Lorenz et al. (2011) Algorithms for Molecular Biology 6:1-26).

In another preferred embodiment of the invention, the above-defined method further comprises filtering nucleic acid sequences to exclude those having features incompatible with miRNAs. This is the case, for instance, if the secondary structure model of the third subsequence does not predict that the first and second subsequence can pair significantly.

In a preferred embodiment of the invention, the above-defined method is implemented for determining if a nucleic acid sequence belongs to miRNA encoded by a plurality of known genomic sequences, wherein the method is performed with a computer comprising a plurality of multi-core processors respectively for each genomic sequence. Advantageously, the method of the invention allows parallel processing using multi-core processors, which increases the computing speed of the method.

In yet another preferred embodiment of the invention, the above-defined method further comprises producing at least one nucleic acid sequence determined as belonging to a miRNA candidate. By way of example the nucleic acid sequence can be produced by chemical synthesis.

The present invention also relates to a method of identification of miRNAs of an organism, comprising:
- sequencing, for example by a Next Generation Sequencing (NGS) technique, a RNA-containing fraction of a cell from an organism having a known genomic sequence to yield RNA reads;
- performing the above-defined method for determining if a nucleic acid sequence belongs to a microRNA (miRNA) candidate

The invention will be further described by the following non-limiting Figures and Example.

### Description of the Figures

Figure 1 represents the biogenesis of Hsa-let-7d miRNA. The nucleotides in bold in the stem-loop pre-miRNA correspond to the mature reference miRNAs Hsa-let-7d-5p and Hsa-let-7d-3p. The various 5p and 3p variants are piled-up with their respective counts and aligned with the genomic MIR sequence where the portions aligning with the reference mature miRNAs are in bold. The dot-bracket notation calculated for this miRNA is represented below the genomic sequence.
Figure 2 illustrates the first step of the method according to the invention taking as an Example a nucleic acid sequence corresponding to Hsa-let-7d-5p. The nucleic acid sequence fully aligns with a first subsequence of the genomic sequence and the reverse complementary sequence of the nucleic acid sequence aligns with 5 errors with a second subsequence of the genomic sequence located in 3' of the first subsequence. The third subsequence of the genomic sequence comprises the first and second subsequence as well as the portion of the genomic sequence between them and has a length equal to or less than 3000 nucleotides. The pivot, *i.e.* the center, of the third subsequence is represented.
Figure 3 represents the first step illustrates the first step of the method according to the invention taking as an Example a nucleic acid sequence corresponding to Hsa-let-7d-3p. The nucleic acid sequence fully aligns with a first subsequence of the genomic sequence and the reverse complementary sequence of the nucleic acid sequence aligns with 8 errors with a second subsequence of the genomic sequence located in 5' of the first subsequence. The third subsequence of the genomic sequence comprises the first and second subsequence as well as the portion of the genomic sequence between them and has a length equal to or less than 3000 nucleotides. The pivot, *i.e.* the center, of the third subsequence is represented
Figure 4 represents the second step of the method according to the invention. A first and second nucleic acid sequences (1 and 2), *e.g.* reads, corresponding to the two most expressed variants of Hsa-let-7d-5p, are fully aligned on the 5' part of a genomic sequence. The reverse complementary of the nucleic acid sequences (1 and 2) are aligned with errors. The two third sequences (1 and 2) share a same pivot (Pivot 1 = Pivot 2).

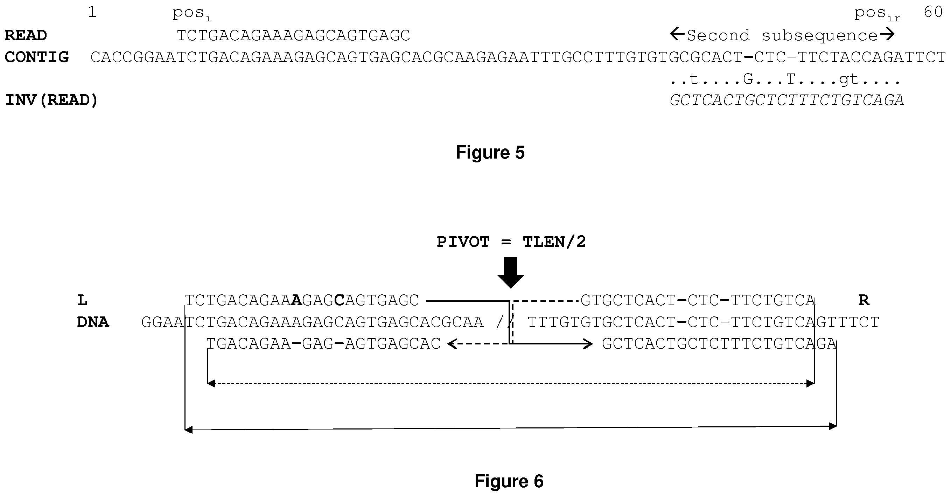

Figure 5 represents a situation where the READ aligns perfectly on the CONTIG with the leftmost position pos*ᵢ*. The BEST ALIGNMENT of REV(READ) has 5 ERRORS, which are relative to the READ: 2 insertions (G and T) and 3 mismatches (G_{CONTIG}->T_{READ} and AC_{CONTIG}->GT_{READ}). Its rightmost position is pos*ᵢᵣ* and the aligned region is the second subsequence. Symbols ".": match; lowercase letters: mismatch; uppercase letters: insertions; "-": deletions. Deletions on the CONTIG have been artificially added for clarity.
Figure 6 illustrates the determination of the PIVOT of *Arabidopsis thaliana* MIR-156a. One read is coming from the miRNA* (linked by the full arrow), and one from the miRNA (linked by the dotted arrow). Both of them have a perfect match on the left (L) or on the right (R), and their reverse complements align with either insertions or deletions, plus a mismatch for the star (T-A in 3'), with a typical overhang of 2nt. TLEN is the length of the interval [leftmost, rightmost] position of READ and INV(READ) alignments (called double-alignments). The two TLENs are different, but they are centered. The center point is the PIVOT.

### EXAMPLE

### Method

The embodiment of the method according to the invention presented hereafter chains two main steps:

### 1. Core step

The core step combines computing of two successive alignments: one perfectly aligning, and one aligning with (at most X) errors as described previously. Those two alignments are called a "double-alignment"..

### 2. Pileup step

Once double-alignments have been produced, miRNA (i.e. MIR and/or miR) candidates are built using a specific pileup process which groups them by PIVOT - *i.e.* the middle-point of the stem-loop as described as pivot or Pivot previously, and in the figures.

An optional step is then applied on the secondary structures predicted by RNAfold, which filters out candidates which stem-loop does not match with a miRNA model.

### 1. Core step

The core step allows qualifying a READ as a mature miRNA (miR) candidate or as being the expression product of a MIR candidate using a CONTIG *(i.e.* a genomic sequence) containing the transcribed sequences yielding the READ. It consists of the following steps (supported by **Figure 5**):
1. Find each position on the CONTIG for which the READ (or the reverse complementary of the READ, *i.e.* INV(READ)) fully aligns *(i.e.* aligns perfectly), thus defining a first subsequence of the CONTIG.
2. If the READ (resp. INV(READ)) aligns perfectly define the position of the BEST ALIGNMENT of INV(READ) (resp. READ) with X ERRORS located in a neighborhood of length T (TLEN = (abs(pos*ᵢᵣ* - pos*ᵢ*)+ 1 ≤ T) of the CONTIG, thereby defining a second subsequence of the CONTIG (pos*ᵢ* is the leftmost position of the first sequence and pos*ᵢᵣ* the rightmost position of the sequence when the first sequence is upstream from the second sequence on the CONTIG oriented in the 5' to 3' position, or pos*ᵢ* is the rightmost position of the first sequence and pos*ᵢᵣ* the leftmost position of the second sequence when the first sequence is downstream from the second sequence; BEG_POS = min(pos*ᵢ* ; pos*ᵢᵣ*).
   The alignment method is based on the Fast Bit-Vector algorithm for Approximate String Matching (Myers (1999) Journal of the ACM 46:395-41).
3. Optionally, for all BEST ALIGNMENTS with X ERRORS realign INV(READ) (resp. READ) on the region defined by the second sequence, by allowing the aligner to treat the pair G-T as a match (G-U Wooble pairing). Dynamic programming with affine gap penalties and asymmetric scoring matrix is used to take Wooble pairs into account.
4. Store the total number of those double-alignments (perfect and BEST ALIGNMENTS) having less or equal than X errors and keep (store) a maximum of MAXS of such alignments (MAXS > 0).

### 2. Pileup step

This section describes how, after having computed and stored the total number of double-alignments as described above, the METHOD proceeds with finding regions on the CONTIG where alignments aggregate, and shape them up to form those pileups.

The fundamental *criterion* is the PIVOT, illustrated by **Figure 6****.**

The PIVOT traduces the symmetrical nature of the folded molecule in regards to the miRNA complex. Any hairpin forming an extra loop in the stem-loop will be neutral from a linear (unfolded) standpoint. This fundamental property is used to cross-validate a miRNA if a sufficient number of pairs of alignments *(i.e.* double-alignments of READ and INV(READ)) share a same pivot and/or if READS of pairs of alignments sharing a same PIVOT can be found on both sides of the PIVOT.

PIVOT should be mathematically defined as PIVOT = BEG_POS + TLEN/2. However, for computer rounding reason PIVOT = 2*BEG_POS + TLEN is retained.

The PILEUP STEP is more precisely defined as:
1. All double-alignments are grouped with respect to the following *criteria*:
   a. All double-alignments have the same PIVOT.
   b. Double-alignments of a same kind are retained. Double-alignments of a same kind are such that they are aligned to the same strand of the genomic sequence. In other words, the READS of double-alignments of a same kind either all fully align or all align with errors. Besides, the READS of double-alignments of a same kind may be found on either side of the PIVOT. Double-alignments of a same kind for which all the READS are on the same side of the PIVOT define a single or M group. Double-alignments of a same kind for which READS are found on both sides of the PIVOT define a double or M/M* group.
   c. Double-alignments of same kind (as described above), ordered by increasing position on the CONTIG, must possibly overlap according to:
      i. a maximum overhang length (configurable: default 4)
      ii. a maximum pileup length (configurable: default 30)
2. The following groups are discarded from candidates:
   a. Having less than *d* double-alignments in groups wherein perfect alignments can be found on both sides of the PIVOT *(i.e.* double or M/M* groups) (configurable: default 20)
   b. Having less than s double-alignments in groups wherein perfect alignments are only found on one side of the PIVOT (*i.e.* single or M groups) (configurable: default 30)
3. Each retained group defines a MIR candidate which can comprise one or two miR candidates depending of perfect alignments are found on only one side of the PIVOT or on both sides of the PIVOT.

### 3. Optional step

1. For candidate groups an RNAfold call is performed, and the MFE (Minimum Free Energy) as well as the secondary structure in dot-bracket notation is stored.
2. An additional filter on the secondary structures computed is performed. Group candidates not passing this filter are pruned off.

### Results

The micro-RNA field is still a research investigation field. Biogenesis is partially known, and validating miRNAs is a complex process. They are therefore not massively abundant in databases.

For assessing the accuracy of the METHOD, the inventors focused on the *Arabidopsis thaliana* (abbreviated as ath) species, which is among the best-documented model organism for mRNAs.

In the following, *Mirbase release 21* (Griffiths-Jones et al. (2006) Nucleic Acids Research, 34(Database issue):D140-4) is relied on as reference database. As a complement, some datasets taken from Breakfield et al. (2012) Genome Research, 22:163-176, which is one of the most comprehensive study in the field, are also relied on.

The following datasets are analyzed:
1. A Positive control: ath_all.
2. A Negative control: Longitudinal_neg
3. A Complete Next-Generation Sequence (NGS) dataset: Longitudinal_raw

The METHOD of the invention is compared against two of the most complete and accurate tools freely available and found to be useable:
- miRDeep2 (Friedlander et al. (2012) Nucleic Acids Research 40:37-2) - version 2.0.0.7
- miRPlant (An et al. (2014) Nucleic Acids Research 41:727-737) - version V4

### 1. Positive Control (ath all)

The positive control has been chosen as follows:
- Mirbase 21 is composed of 325 miRNA-encoding genes (MIRs) from *Arabidopsis thaliana,* totaling 427 mature miRNAs (miRs);
- 26 out these 325 MIRs do not have any NGS support evidence;
- Among the 299 MIRs remaining :
   ∘ 78 are tagged as "verified", totaling 135 miRs,
   ∘ 247 are tagged as non-verified, totaling 292 miRs;
- The ath-all dataset is the cumulated set of NGS reads present in the mirbase records, used to assess the evidence of such 78 verified MIRs. ath-all is composed of 1 236 275 NGS reads amounting to 1 447 unique (deduplicated) reads.

The METHOD of the invention yielded 82 MIR candidates allowing identifying 62 MIRs out of 78 possible (80% identification rate) and 109 miRs out of 135 possible, (80.7% identification rate).

In contrast:
- miRPlant yielded 74 MIR candidates, allowing identifying 63 MIRs out of 78 possible (80.7% identification rate);
- miRDeep2 yielded 45 MIR candidates, allowing identifying 37 MIRs out of 78 possible (47.4% identification rate).

The sensitivity of the general METHOD according to the invention is thus equivalent to a plant-specific reference method and improved with respect to a general reference method.

### 2. Negative Control (Longitudinal neg)

The negative control set is a set of NGS reads being potentially smallRNAs, but not microRNAs. This set has been chosen as follows:
- Starting from the Complete Dataset Longitudinal_raw (see below), all reads were perfectly aligned against the RNA family (RFAM) database (Burge et al. (2013) Nucleic Acids Research 41 (Database issue):D226-32), and only aligned reads against records of RFAM not annotated as microRNA (tRNA, rRNA, so-RNA, etc.) were kept;
- Longitudinal_neg is thus composed of 2 171 760 NGS non-microRNA reads amounting to 121 466 unique (deduplicated) non-microRNA reads.

The method according to the invention only identified 5 MIR candidates, thereby showing its strong specificity.

### 3. Complete NGS dataset (Longitudinal raw)

The complete Dataset is composed of the SRR218085.fast, SRR218086.fastq, SRR218087.fastq, and SRR218088.fastq files downloadable from the NCBI SRA archive and corresponding to *Arabidopsis thaliana* RNA reads from root tissues (Breakfield et al. (2012) Genome Research 22:163-176).

Longitudinal_raw is thus composed of 82 058 247 NGS reads amounting to 8 976 123 unique (deduplicated) reads.

The comparative results are shown in the following **Table 1.**

**Table 1: Comparison with miRDeep2 and miRPlant**

| **LONG_RAW** | **miRDeep2** | | **miRPlant** | **METHOD** | |
|---|---|---|---|---|---|
| | *no_rand* | *rand* | | *NO_RF* | *RF* |
| **# Candidates** | 625 | 781 | 1981 | 2786 | 1897 |
| **# MIRs Found *(versus 325 mirbase MIRs)*** | 91 | 95 | 98 | 143 | 141 |
| **# novels** | 511 | 668 | 1882 | 2588 | 1708 |
| **Runtime (*in min.)*** | 1500 | 11520 | 1200 | 10 | |

**Table 1** compares the MIR candidates found with miRDeep2 (*no_rand*: no randomization; *rand*: randomization), miRPlant and the METHOD according to the invention (*NO_RF*: No RNAfold filter: *RF*: RNAfold filter activated), as well as runtimes.

miRDeep2 being more stringent in candidates kept - as well as more limited than miRPlant in precursor lengths windowing - it has the lowest number of candidates and putative novels. When activating the RNAfold filter (RF), the METHOD of the invention provides an equivalent number of candidates and novel candidates than miRPlant, while providing with an approximate 50% better recall than the two other tools (141 versus 91-98).

Taking away miRDeep2 in randomization mode (runtime of 8 days), the METHOD of the invention is two orders of magnitude faster (same hardware, multi-core departmental Linux server).

This 100x speedup is due to the 'by-design' reduction of complexity by the 'double-alignment' approach according to the invention. As a matter of example, miRDeep2 (no_rand) computes 1.1M+ RNA folding, while the METHOD computes 11,000 RNA folding for all candidates, which is a ratio of 1/100. While the overall runtime is not completely linked to the number of RNAfold calls, it is a significant indicator of the breakthrough provided by the METHOD. As a side effect of this, RF computation is insignificant.

## Claims

1. A computer-implemented method for determining if a nucleic acid sequence belongs to a microRNA (miRNA) candidate encoded by at least one known genomic sequence, comprising:
a) determining if the nucleic acid sequence or the reverse complementary sequence thereof can fully align with a first subsequence of the known genomic sequence;
b) then,
- if the nucleic acid sequence fully aligns with the first subsequence of the known genomic sequence, determining if the reverse complementary sequence of the nucleic acid sequence can align with a second subsequence of the known genomic sequence with X error(s) at the most, or
- if the reverse complementary nucleic acid sequence of the nucleic acid sequence fully aligns with the first subsequence of the known genomic sequence, determining if the nucleic acid sequence can align with a second subsequence of the known genomic sequence with X error(s) at the most;
wherein X is an integer from 0 to 0.6L, wherein L is the length of the nucleic acid sequence, and a third subsequence of the known genomic sequence which consists of the first and second subsequences and of the portion of the known genomic sequence between the first and second subsequences has a length T, wherein T ≤ 3000 nucleotides;
c) whereby, if conditions a) and b) are met, the nucleic acid sequence is determined to belong to a miRNA candidate.

2. The method of claim 1, further comprising:
- providing at least two nucleic acid sequences for which conditions a) and b) are met;
- determining that the at least two nucleic acid sequences belong to a same miRNA candidate if their respective third subsequences of the known genomic sequences share a same pivot.

3. The method of claim 2, wherein the at least two nucleic acid sequences mutually overlap.

4. The method according to claim 2, wherein one of the at least two nucleic acid sequences fully aligns with a subsequence of the known genomic sequence while the reverse complementary of the other of the at least two nucleic acid sequences fully aligns with a subsequence of the known genomic sequence.

5. The method of any of claims 1 to 4, wherein alignment with the second subsequence in step b) takes wobble pairing into account.

6. The method of any of claims 1 to 5, wherein X ≤ 7.

7. The method of any of claims 1 to 6, wherein 30 ≤ T ≤ 350.

8. The method of any of claims 1 to 6, wherein 350 ≤ T ≤ 3000.

9. The method of any of claims 1 to 8, wherein the known genomic sequence is a contig, a scaffold or a chromosome.

10. The method of any of claims 1 to 9, wherein the nucleic acid sequence is a sequencing RNA read, in particular a next generation sequencing (NGS) RNA read.

11. The method of any of claims 1 to 10, further comprising filtering nucleic acid sequences to exclude those having features incompatible with miRNAs.

12. The method according to any of claims 1 to 11, further comprising modeling the secondary structure of the third subsequence of the known genomic sequence consisting of the respective first and second subsequences and of the portion of the known genomic sequence between the first and second subsequences by minimizing nucleotide-pairing free-energy.

13. The method of any of claims 1 to 12, for determining if a nucleic acid sequence belongs to miRNA encoded by a plurality of known genomic sequences, wherein the method is performed with a plurality of multi-core processors respectively for each genomic sequence.

14. The method of any of claims 1 to 13, further comprising producing at least one nucleic acid sequence determined as belonging to a miRNA candidate.

15. A method of identification of miRNAs of an organism, comprising:
- sequencing a RNA-containing fraction of a cell from an organism having a known genomic sequence to yield RNA reads;
- performing the method according to any of claims 1 to 14.

## Patentansprüche

1. Computerimplementiertes Verfahren zur Bestimmung, ob eine Nukleinsäuresequenz zu einem MikroRNA(miRNA)-Kandidaten gehört, der durch mindestens eine bekannte Genomsequenz codiert wird, umfassend:
a) Bestimmung, ob die Nukleinsäuresequenz oder die reverse komplementäre Sequenz davon mit einer ersten Untersequenz der bekannten Genomsequenz vollkommen übereinstimmt,
b) danach,
- wenn die Nukleinsäuresequenz mit der ersten Untersequenz der bekannten Genomsequenz vollkommen übereinstimmt, Bestimmung, ob die reverse komplementäre Sequenz der Nukleinsäuresequenz mit einer zweiten Untersequenz der bekannten Genomsequenz mit höchstens X Fehlern übereinstimmt, oder
- wenn die reverse komplementäre Nukleinsäuresequenz mit der ersten Untersequenz der bekannten Genomsequenz vollkommen übereinstimmt, Bestimmung, ob die Nukleinsäuresequenz mit einer zweiten Untersequenz der bekannten Genomsequenz mit höchstens X Fehlern übereinstimmt,
wobei X eine ganze Zahl zwischen 0 und 0,6L ist, wobei L die Länge der Nukleinsäuresequenz ist, und eine dritte Untersequenz der bekannten Genomsequenz, die aus der ersten Untersequenz und der zweiten Untersequenz, sowie dem Abschnitt der bekannten Genomsequenz zwischen der ersten Untersequenz und der zweiten Untersequenz besteht, eine Länge T hat, wobei T ≤ 3000 Nukleotide beträgt,
c) wobei, wenn die Bedingungen a) und b) erfüllt werden, die Nukleinsäuresequenz als zu einem miRNA-Kandidaten gehörend bestimmt ist.

2. Verfahren nach Patentanspruch 1, außerdem unmfassend:
- Vorlage von mindestens zwei Nukleinsäuresequenzen, die die Bedingungen a) und b) erfüllen,
- Bestimmung, dass die mindestens zwei Nukleinsäuresequenzen zu demselben miRNA-Kandidaten gehören, wenn ihre jeweiligen dritten Untersequenzen der bekannten Genomsequenzen denselben Drehpunkt miteinander gemeinsam haben.

3. Verfahren nach Patentanspruch 2, in dem die mindestens zwei Nukleinsäuresequenzen einander überlappen.

4. Verfahren nach Patentanspruch 2, in dem eine der mindestens zwei Nukleinsäuresequenzen mit einer Untersequenz der bekannten Genomsequenz vollkommen übereinstimmt, während die reverse komplementäre Nukleinsäuresequenz der anderen der mindestens zwei Nukleinsäuresequenzen mit einer Untersequenz der bekannten Genomsequenz vollkommen übereinstimmt.

5. Verfahren nach irgendeinem der Patentansprüche 1 bis 4, in dem Übereinstimmung mit der zweiten Untersequenz in Schritt (b) Wobble-Paarungen berücksichtigt.

6. Verfahren nach irgendeinem der Patentansprüche 1 bis 5, in dem X ≤ 7.

7. Verfahren nach irgendeinem der Patentansprüche 1 bis 6, in dem 30 ≤ T ≤ 350.

8. Verfahren nach irgendeinem der Patentansprüche 1 bis 6, in dem 350 ≤ T ≤ 3000.

9. Verfahren nach irgendeinem der Patentansprüche 1 bis 8, in dem die bekannte Genomsequenz ein Contig, ein Kerngerüst oder ein Chromosom ist.

10. Verfahren nach irgendeinem der Patentansprüche 1 bis 9, in dem die Nukleinsäuresequenz ein Sequenzierungs-RNA-Read, insbesondere ein RNA-Read aus Hochdurchsatz-Sequenzierung ist.

11. Verfahren nach irgendeinem der Patentansprüche 1 bis 10, außerdem Filterung der Nukleinsäuresequenzen umfassend, um diejenigen auszuschließen, die mit miRNA inkompatible Merkmale haben.

12. Verfahren nach irgendeinem der Patentansprüche 1 bis 11, außerdem Modellierung der Sekundärstruktur der dritten Untersequenz der bekannten Genomsequenz, die aus der ersten Untersequenz und der zweiten Untersequenz, sowie dem Abschnitt der bekannten Genomsequenz zwischen der ersten Untersequenz und der zweiten Untersequenz besteht, durch Minimisierung der freien Energie der Nukleotidpaarung umfassend.

13. Verfahren nach irgendeinem der Patentansprüche 1 bis 12 zur Bestimmung, ob eine Nukleinsäuresequenz zu miRNA gehört, die durch mehrere bekannte Genomsequenzen codiert wird, wobei das Verfahren mit mehreren Mehrkernprozessoren jeweils für jede Genomsequenz ausgeführt wird.

14. Verfahren nach irgendeinem der Patentansprüche 1 bis 13, außerdem umfassend, mindestens eine Nukleinsäuresequenz zu erzeugen, die als zu einem miRNA-Kandidaten gehörend bestimmt wurde.

15. Verfahren zur Identifizierung von miRNA eines Organismus, umfassend:
- Sequenzierung einer RNA-haltigen Zellfraktion eines Organismus mit bekannter Genomsequenz zur Erstellung von RNA-Reads,
- Ausführung des Verfahrens nach irgendeinem der Patentansprüche 1 bis 14.

## Revendications

1. Procédé mis en œuvre par ordinateur pour déterminer si une séquence d'acide nucléique appartient à un candidat microARN (miARN) codé par au moins une séquence génomique connue, comprenant :
a) déterminer si la séquence d'acide nucléique ou la séquence complémentaire inverse de celle-ci peut complètement s'aligner avec une première sous-séquence de la séquence génomique connue ;
b) puis
- si la séquence d'acide nucléique s'aligne complètement avec la première sous-séquence de la séquence génomique connue, déterminer si la séquence complémentaire inverse de la séquence d'acide nucléique peut s'aligner avec une deuxième sous-séquence de la séquence génomique connue avec au plus X erreur(s), ou
- si la séquence d'acide nucléique complémentaire inverse de la séquence d'acide nucléique s'aligne complètement avec la première sous-séquence de la séquence génomique connue, déterminer si la séquence d'acide nucléique peut s'aligner avec une deuxième sous-séquence de la séquence génomique connue avec au plus X erreur(s) ;
dans lequel X est un entier de 0 à 0,6L, où L est la longueur de la séquence d'acide nucléique, et une troisième sous-séquence de la séquence génomique connue qui est constituée des première et deuxième sous-séquences et de la partie de la séquence génomique connue entre les première et deuxième sous-séquences a une longueur T, où T ≤ 3000 nucléotides ;
c) en déduire, si les conditions a) et b) sont remplies, que la séquence d'acide nucléique est déterminée comme appartenant à un candidat miARN.

2. Procédé selon la revendication 1, comprenant en outre :
- fournir au moins deux séquences d'acide nucléique pour lesquelles les conditions a) et b) sont remplies ;
- déterminer que les au moins deux séquences d'acide nucléique appartiennent à un même candidat miARN si leurs troisièmes sous-séquences des séquences génomiques connues respectives partagent un même pivot.

3. Procédé selon la revendication 2, dans lequel les au moins deux séquences d'acide nucléique se recouvrent mutuellement.

4. Procédé selon la revendication 2, dans lequel une des au moins deux séquences d'acide nucléique s'aligne complètement avec une sous-séquence de la séquence génomique connue alors que la complémentaire inverse de l'autre des au moins deux séquences d'acide nucléique s'aligne complètement avec une sous-séquence de la séquence génomique connue.

5. Procédé selon l'une quelconque des revendications 1 à 4, dans lequel l'alignement avec la deuxième sous-séquence dans l'étape b) prend en compte l'appariement par flottement.

6. Procédé selon l'une quelconque des revendications 1 à 5, dans lequel X ≤ 7.

7. Procédé selon l'une quelconque des revendications 1 à 6, dans lequel 30 ≤ T ≤ 350.

8. Procédé selon l'une quelconque des revendications 1 à 6, dans lequel 350 ≤ T ≤ 3000.

9. Procédé selon l'une quelconque des revendications 1 à 8, dans lequel la séquence génomique connue est un contig, un scaffold ou un chromosome.

10. Procédé selon l'une quelconque des revendications 1 à 9, dans lequel la séquence d'acide nucléique est une séquence de lecture d'ARN issue d'un séquençage, en particulier une séquence de lecture d'ARN issue d'un séquençage de nouvelle génération (NGS).

11. Procédé selon l'une quelconque des revendications 1 à 10, comprenant en outre le filtrage des séquences d'acide nucléique pour exclure celles ayant des caractéristiques incompatibles avec les miARN.

12. Procédé selon l'une quelconque des revendications 1 à 11, comprenant en outre la modélisation de la structure secondaire de la troisième sous-séquence de la séquence génomique connue constituée des première et deuxième sous-séquences respectives et de la partie de la séquence génomique connue entre les première et deuxième sous-séquences en minimisant l'énergie libre d'appariement des nucléotides.

13. Procédé selon l'une quelconque des revendications 1 à 12, pour déterminer si une séquence d'acide nucléique appartient à un miARN codé par une pluralité de séquences génomiques connues, dans lequel le procédé est mis en œuvre avec une pluralité de processeurs multi-cœurs respectivement pour chaque séquence génomique.

14. Procédé selon l'une quelconque des revendications 1 à 13, comprenant en outre la production d'au moins une séquence d'acide nucléique déterminée comme appartenant à un candidat miARN.

15. Procédé d'identification de miARN d'un organisme, comprenant :
- séquencer une fraction contenant des ARN d'une cellule d'un organisme ayant une séquence génomique connue pour donner des séquences de lecture d'ARN ;
- mettre en œuvre le procédé selon l'une quelconque des revendications 1 à 14.


### SEQUENCE LISTING

<110> BIOFACET SAS
<120> COMPUTER-IMPLEMENTED METHOD FOR THE IDENTIFICATION OF MICRO-RNAs
<130> B000050EP
<160> 21
<170> PatentIn version 3.5
<210> 1
   <211> 87
   <212> DNA
   <213> Homo sapiens
<400> 1
   cctaggaaga ggtagtaggt tgcatagttt tagggcaggg attttgccca caaggaggta        60
   actatacgac ctgctgcctt tcttagg                                            87
<210> 2
   <211> 87
   <212> RNA
   <213> Homo sapiens
<400> 2
   ccuaggaaga gguaguaggu ugcauaguuu uagggcaggg auuuugccca caaggaggua 60
   acuauacgac cugcugccuu ucuuagg 87
<210> 3
   <211> 23
   <212> RNA
   <213> Homo sapiens
<400> 3
   aagagguagu agguugcaua guu 23
<210> 4
   <211> 19
   <212> RNA
   <213> Homo sapiens
<400> 4
   agagguagua gguugcaua 19
<210> 5
   <211> 24
   <212> RNA
   <213> Homo sapiens
<400> 5
   agagguagua gguugcauag uuuu 24
<210> 6
   <211> 20
   <212> RNA
   <213> Homo sapiens
<400> 6
   agagguagua gguugcauag 20
<210> 7
   <211> 18
   <212> RNA
   <213> Homo sapiens
<400> 7
   agagguagua gguugcau 18
<210> 8
   <211> 21
   <212> RNA
   <213> Homo sapiens
<400> 8
   gagguaguag guugcauagu u 21
<210> 9
   <211> 20
   <212> RNA
   <213> Homo sapiens
<400> 9
   gagguaguag guugcauagu 20
<210> 10
   <211> 21
   <212> RNA
   <213> Homo sapiens
<400> 10
   cuauacgacc ugcugccuuu c 21
<210> 11
   <211> 21
   <212> RNA
   <213> Homo sapiens
<400> 11
   uauacgaccu gcugccuuuc u 21
<210> 12
   <211> 19
   <212> RNA
   <213> Artificial
<220>
   <223> Reverse complementary of Hsa-let-7d-5p
<400> 12
   uaugcaaccu acuaccucu 19
<210> 13
   <211> 21
   <212> RNA
   <213> Artificial
<220>
   <223> Reverse complementary of Hsa-let-7d-3p
<400> 13
   gaaaggcggc gggucguaua g 21
<210> 14
   <211> 23
   <212> RNA
   <213> Homo sapiens
<400> 14
   aagagguagu agguugcaua guu 23
<210> 15
   <211> 23
   <212> RNA
   <213> Artificial
<220>
   <223> Reverse complementary of Hsa-let-7d-5p
<400> 15
   aacuaugcaa ccuacuaccu cuu 23
<210> 16
   <211> 78
   <212> DNA
   <213> Artificial
<220>
   <223> CONTIG
<400> 16
   caccggaatc tgacagaaag agcagtgagc acgcaagaga atttgccttt gtgtgcgcac 60
   tctcttctac cagattct 78
<210> 17
   <211> 22
   <212> DNA
   <213> Artificial
<220>
   <223> READ
<400> 17
   tctgacagaa agagcagtga gc 22
<210> 18
   <211> 22
   <212> DNA
   <213> Artificial
<220>
   <223> INV(READ)
<400> 18
   gctcactgct ctttctgtca ga 22
<210> 19
   <211> 63
   <212> DNA
   <213> Artificial
<220>
   <223> CONTIG
<400> 19
   ggaatctgac agaaagagca gtgagcacgc aatttgtgtg ctcactctct tctgtcagtt 60
   tct 63
<210> 20
   <211> 20
   <212> DNA
   <213> Artificial
<220>
   <223> READ
<400> 20
   gtgctcactc tcttctgtca 20
<210> 21
   <211> 20
   <212> DNA
   <213> Artificial
<220>
   <223> INV(READ)
<400> 21
   tgacagaaga gagtgagcac 20